# EUROPÄISCHE PATENTSCHRIFT

(11) **EP 3 286 325 B1**
(45) Veröffentlichungstag und Bekanntmachung des Hinweises auf die Patenterteilung: **13.07.2022**
(21) Anmeldenummer: 16711778.7
(22) Anmeldetag: 26.02.2016
(51) Int. Cl.: C12Q 1/6806

(54) **VERFAHREN UND TESTKIT ZUR SCHNELLEN ISOLIERUNG VON NUKLEINSÄUREN MITTELS RAUER OBERFLÄCHEN**
METHOD AND TEST KIT FOR RAPID ISOLATION OF NUCLEIC ACIDS USING ROUGH SURFACES
PROCÉDÉ ET KIT D'ESSAI POUR L'ISOLATION RAPIDE D'ACIDES NUCLÉIQUES AU MOYEN DE SURFACES RUGUEUSES

(30) Priorität: 23.04.2015 DE 102015207481; 19.06.2015 DE 102015211393; 19.06.2015 DE 102015211394
(43) Veröffentlichungstag der Anmeldung: 28.02.2018
(73) Patentinhaber: IST Innuscreen GmbH, 13125 Berlin (DE)
(72) Erfinder: HILLEBRAND, Timo, 15366 Hoppegarten (DE); STROH, Thorsten, 10713 Berlin (DE)
(74) Vertreter: Wehlan, Helmut
(86) Internationale Anmeldenummer: PCT/EP2016/054178
(87) Internationale Veröffentlichungsnummer: WO 2016/169677

(56) Entgegenhaltungen:
- EP-A1- 1 690 938
- WO-A1-01/62976
- WO-A1-02/38758
- DE-A1-102005 053 463
- DE-A1-102005 059 217
- VOGELSTEIN B ET AL: "PREPARATIVE AND ANALYTICAL PURIFICATION OF DNA FROM AGAROSE", PROCEEDINGS OF THE NATIONAL ACADEMY OF SCIENCES, NATIONAL ACADEMY OF SCIENCES, US, Bd. 76, Nr. 2, 1. Februar 1979 (1979-02-01), Seiten 615-619, XP002906050, ISSN: 0027-8424, DOI: 10.1073/PNAS.76.2.615

## Beschreibung

Gegenstand der Erfindung ist ein völlig neuartiges, universelles, stark vereinfachtes sowie extrem schnelles Verfahren zur Isolierung von Nukleinsäuren aus Nukleinsäuren enthaltenden unterschiedlichsten Ausgangsmaterialien, welches sowohl eine sehr hohe Qualität der zu isolierenden Nukleinsäuren garantiert als auch die Isolierung extrem hoher Nukleinsäure-Ausbeuten ermöglicht. Das Verfahren kann sowohl manuell im Labor, unter Feldbedingungen als auch vollständig automatisiert durchgeführt werden. Es basiert auf der Anbindung von Nukleinsäuren an eine nicht-glatte Oberfläche.

Unter klassischen Bedingungen erfolgt die Isolierung von DNA aus Zellen und Geweben dadurch, dass die Nukleinsäuren enthaltenden Ausgangsmaterialien unter stark denaturierenden und reduzierenden Bedingungen, teilweise auch unter Verwendung von proteinabbauenden Enzymen aufgeschlossen, die austretenden Nukleinsäurefraktionen über Phenol-/ Chloroform- Extraktionsschritte gereinigt und die Nukleinsäuren mittels Dialyse oder Ethanolpräzipitation aus der wässrigen Phase gewonnen werden (Sambrook, J., Fritsch, E.F. und Maniatis, T., 1989, CSH, "Molecular Cloning").

Diese "klassischen Verfahren" zur Isolierung von Nukleinsäuren aus Zellen und besonders aus Geweben sind sehr zeitaufwendig (teilweise länger als 48 h), erfordern einen erheblichen apparativen Aufwand und sind darüber hinaus auch nicht unter Feldbedingungen realisierbar. Außerdem sind solche Methoden auf Grund der verwendeten Chemikalien wie Phenol und Chloroform in einem nicht geringen Maße gesundheitsgefährdend.

Die nächste Verfahrensgeneration zur Isolierung von Nukleinsäuren basiert auf einer von Vogelstein und Gillespie (Proc. Natl. Acad. Sci. USA, 1979, 76, 615 - 619) entwickelten und erstmals beschriebenen Methode zur präparativen und analytischen Reinigung von DNA-Fragmenten aus Agarosegelen. Die Methode kombiniert die Auflösung der die zu isolierende DNA- Bande enthaltenden Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und danach von den Trägerpartikeln abgelöst. Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet und ist letztlich die Basis für fast alle kommerziell verfügbaren Kits zur manuellen wie auch automatisierten Isolierung von Nukleinsäuren. Darüber hinaus gibt es eine Vielzahl von Patenten und Veröffentlichungen, die sich auf das Basisprinzip der Isolierung von Nukleinsäuren beziehen, welches von Vogelstein und Gillespie erstmals publiziert wurde und ggf. weitere Vorteile innehaben. Diese Varianten betreffen sowohl die Verwendung unterschiedlicher mineralischer Trägermaterialien als auch die Art der für die Bindung der Nukleinsäuren eingesetzten Puffer. Beispiele sind u.a. die Bindung von Nukleinsäuren an mineralische Träger unter Anwesenheit von Lösungen unterschiedlicher chaotroper Salze, bei welchen als Trägermaterial feingemahlene Glaspulver (BIO 101, La Jolla, CA), Diatomenerden (Fa.Sigma) oder auch Silicagele bzw. Silcasuspensionen oder Glasfaserfilter oder mineralische Erden (DE 41 39 664 A1; US 5,234,809; WO-A 95/34569 DE 4321904; DE 20207793) zum Einsatz kommen. All diese Schriften basieren auf der Anbindung von Nukleinsäuren an ein mineralisches Trägermaterial auf der Basis von Glas oder Silizium unter Anwesenheit chaotroper Salzlösungen. In neueren Patentschriften wird offenbart, dass für die Adsorption von Nukleinsäuren an die dem Fachmann bekannten und eingesetzten mineralischen Materialien auch sogenannte antichaotrope Salze als Bestandteil von Lyse-/ Bindungspuffer-Systemen sehr effizient und erfolgreich eingesetzt werden können (EP 1135479).

Zusammenfassend kann man den Stand der Technik also dahingehend beschreiben, dass Nukleinsäuren an mineralische Materialien in Anwesenheit von Puffern, die chaotrope oder antichaotrope Salze enthalten oder auch in Anwesenheit von Puffern die Mischungen chaotroper und antichaotroper Salze enthalten, binden und auf diesem Wege dann auch isoliert werden können. Dabei gibt es auch Vorzugsvarianten, bei denen zusätzlich aliphatische Alkohole zur Bindungsvermittlung eingesetzt werden. Dem Fachmann ist auch bekannt, dass alle gängigen kommerziellen Produkte zur Isolierung und Aufreinigung von Nukleinsäuren auf dieser Grundlage basieren. Die eingesetzten mineralischen Träger liegen dabei in Form von losen Schüttungen, in Form von Filtermembranen oder auch in Form von Suspensionen vor. Für die Durchführung von automatisierten Extraktionsabläufen werden häufig paramagnetische oder magnetische Partikel eingesetzt. Dabei handelt es sich z.B. um silikatische Materialien, die einen magnetischen oder paramagnetischen Kern besitzen oder aber auch um Eisenoxidpartikel, deren Oberfläche so modifiziert wird, dass diese die für die Anbindung der Nukleinsäuren notwendigen Funktionalitäten tragen. Die Extraktionsabläufe zur Isolierung von Nukleinsäuren basieren dabei auch nach prinzipiell gleichen Schemata: Lyse der Ausgangsprobe zur Freisetzung der Nukleinsäure, binden der Nukleinsäure an das entsprechende mineralische Trägermaterial, waschen der gebundenen Nukleinsäure, Trocknung des Trägermaterials und finale Elution der gebundenen Nukleinsäure vom Trägermaterial. Auch wenn sich diese Verfahren bewährt haben, gibt es doch auch eine Reihe von Nachteilen. So ist die Bindungskapazität der eingesetzten Materialien begrenzt, insbesondere bei der Verwendung von Spinfiltermembranen. Wenn die Ausgangsprobe zu viel Nukleinsäure enthält, verstopfen Membranen auch oftmals. Die automatisierten Prozessabläufe unter Verwendung von magnetischen Partikeln sind relativ aufwendig und benötigen je nach Anwendung auch einen relativ hohen Zeitaufwand. Die einfache Durchführung einer Nukleinsäure-Isolierung unter Feldbedingungen ist nicht gegeben.

### Aufgabe der Erfindung

Der Erfindung lag deshalb die Aufgabe zu Grunde, die Nachteile der im Stand der Technik beschriebenen technischen Lösungen zu beseitigen.

### Lösung der Aufgabe

Die Aufgabe wurde gemäß den Merkmalen der Patentansprüche gelöst. Gemäß Anspruch 1 wird ein Verfahren zur Isolierung von Nukleinsäuren aus wässrigen, Nukleinsäure-enthaltenden Proben beschrieben, wobei eine wässrige Lösung, die freie oder durch Lyse freigesetzte Nukleinsäuren enthält, vor oder nach Senkung der Polarität der wässrigen Lösung mit einer festen Phase, die eine raue Oberfläche aufweist, in Kontakt gebracht wird, wobei die Nukleinsäuren an der festen Phase präzipitieren - und damit an die feste Phase angebunden werden - und anschließend aus dieser wässrigen Lösung mit der festen Phase entfernt werden. Der Begriff "raue Oberfläche" beschreibt im Sinne der Erfindung, dass die Rauheit der Oberfläche durch Berühren oder durch Ansicht der Oberfläche erkennbar ist. Der Begriff "vor oder nach Senkung der Polarität der wässrigen Lösung" beschreibt die Möglichkeit, dass die wässrige Lösung sowohl zuerst mit der festen Phase in Kontakt gebracht werden kann und dann die Polarität des Wassers gesenkt wird als auch die Möglichkeit, dass zuerst die Polarität des Wassers gesenkt wird und dann der Kontakt mit der festen Phase erfolgt. Bei zuerst genanntem erfolgt die Präzipitation der Nukleinsäuren an die festen Phase erst, wenn die Polarität des Wassers gesenkt wird - in der Regel durch Zugabe eines Alkohols. Bei der anderen Variante erfolgt die Präzipitation der Nukleinsäuren an die festen Phase sofort mit dem In-Kontakt-Bringen. Werden wasserunlösliche organische Lösungsmittel verwendet, so muss die Mischung bewegt werden, damit es an der festen Phase zur Präzipitation der Nukleinsäuren kommt. Dies erfolgt vorzugsweise durch Bewegen der festen Phase innerhalb der Mischung.

Die Ansprüche 2 bis 8 zeigen bevorzugte Ausführungsformen der beanspruchten Erfindung. Gegenstand der Erfindung ist auch ein Testkit zur Durchführung des Verfahrens sowie Geräte dafür.

Erfindungsgemäß wurde ein Verfahren zur Isolierung von Nukleinsäuren bereitgestellt, welches deutlich einfacher in der Durchführung ist als die bekannten Verfahren; welches es erlaubt, eine Nukleinsäure-Isolierung auch unter Feldbedingungen (und durch Nichtspezialisten) durchführen zu können, welches extrem schnell in der Durchführung ist - insbesondere im Kontext mit einer automatisierten Extraktion - und welches es erlaubt, extrem hohe Ausbeuten an Nukleinsäuren mit hoher Qualität zu isolieren.

Der Erfindung lag folgende Beobachtung zu Grunde. Wenn man eine Nukleinsäure enthaltende Probe mit Extraktionsreagenzien von kommerziell verfügbaren Kits (z.B. innuPREP Blood DNA Kit/IPC16, Analytik Jena AG; DNeasy Blood & Tissue Kit; Fa. Qiagen) bearbeitet und man anstelle eines mineralischen Trägermaterials ein beliebiges Plastikmaterial mit einer rauen Oberfläche (beispielsweise angerautes Polypropylen) einsetzt und dieses in Kontakt mit der in Bearbeitung befindlichen Probe bringt, bindet Nukleinsäure an das Plastikmaterial.

Der Begriff "raue Oberfläche" ist so zu verstehen, dass durch Berühren oder durch Ansicht der Oberfläche erkennbar ist, dass diese nicht glatt ist. Dabei kann es sich aber auch um eine Oberfläche handeln, die eine Struktur aufweist (z.B. Rillen). Durch diese Struktur ist die Glattheit der Oberfläche aufgehoben, auch wenn die Struktur, also die Rillen, selbst glatt sein kann. Falls durch Ansicht oder Berühren der Oberfläche nicht erkennbar ist, ob eine Oberfläche glatt oder rau ist, kann ein Test durchgeführt werden, bei dem ein Laserstrahl auf diese Oberfläche gerichtet wird. Bei einer glatten Oberfläche wird der Laser nur in Hauptrichtung an der Oberfläche reflektiert. Bei rauen Oberflächen erfolgt eine Streuung in alle Raumrichtungen. Ein solcher Test ist auf der Web-Seite der Universität Kiel beschrieben worden (http://www.tf.unikiel.de/matwis/amat/semitech en/kap 3/illustr/oberflaechenstrukture.pdf), Seite 7.

Die nachfolgenden Schritte des Waschens können mit bekannten alkoholhaltigen Waschpuffern oder auch nur mit einem Alkohol durchgeführt werden, ebenso können für die Elution bekannte Niedrigsalzpuffer oder auch Wasser eingesetzt werden. Der einzige Unterschied ist, dass anstelle des mineralischen Trägermaterials ein raues Plastikmaterial eingesetzt wird. Es zeigte sich schon nach den ersten Experimenten, dass damit alle Prozessschritte viel einfacher und schneller umgesetzt werden können. Es zeigt sich auch, dass dieser Effekt mit allen eingesetzten rauen Plastikmaterialien zu beobachten war. Die Besonderheit bei all den durchgeführten Versuchen bestand aber anfangs darin, dass die verwendeten rauen Plastikmaterialien auf einem 3D-Drucker gefertigt wurden. Dadurch wird die Oberfläche nicht glatt, sondern geriffelt - also rau, da sich beim 3D-Druck ein Körper bildet, der schichtförmig aufgebaut ist. Durchgeführt wurden die ersten Experimente dabei unter Nutzung eines Extraktionsautomaten (Thermo Electron), dem sog. Magnetpartikelprozessor KingFisher ml. Es handelt sich dabei um ein Gerät zur Extraktion von Nukleinsäuren mittels magnetischer Partikel. Das Gerät verwendet dabei Plastikkämme, in welche Magnetstäbe einfahren, welche dann in einem walk-away-Prozess Magnetpartikel bewegen und in die für eine Standardextraktion notwendigen Puffer eintauchen. Diese Plastikkämme wurden für das erfindungsgemäße Verfahren zweckentfremdet eingesetzt. Dazu wurden mittels eines 3D-Druckers Hülsen aus verschiedenen Materialien gedruckt, welche dann auf die Plastikkämme aufgesteckt wurden. Als Probe wurden jeweils ca. 1 × 10⁶ NIH 3T3 Zellen verwendet. Die für die Isolierung der Nukleinsäuren eingesetzten Reagenzien wurden dabei z.B. teilweise aus dem kommerziell verfügbaren Extraktionskit (innuPREP Blood DNA Mini Kit/IPC16; Analytik Jena AG) genommen. Der Ablauf der Extraktion folgte folgendem workflow. Unter Verwendung des Lysepuffers (Lysis Solution CBV) sowie von Proteinase K wurden die Zellen bei 60°C für 15 min. lysiert. Während der Lyse wurde die Reaktionsplastik des KingFisher ml mit folgenden Lösungen vorbefüllt:
Kavität 1: 400 µl Isopropanol
Kavität 2: 800 µl Washing Solution LS (aus dem Extraktionskit)
Kavität 3: 800 µl 80% Ethanol
Kavität 4: 800 µl 80% Ethanol
Kavität 5: 200 µl Elution Buffer (aus dem Extraktionskit)

Nach erfolgter Lyse wurde das Lysat (400 µl) in die Kavität 1 zum dort schon vorliegenden Isopropanol gegeben und der Extraktionsprozess gestartet. Bei diesem Prozess bewegen sich die Plastikkämme sukzessive von Kavität 1 bis Kavität 5. Dabei erfolgt in jeder Kavität eine vertikale Bewegung der Plastikkämme in die jeweils vorliegende Pufferlösung. Der Extraktionsprozess basiert dabei auf den klassischen Prinzipien der Nukleinsäure-Isolierung unter Verwendung von magnetischen Partikeln. Es werden aber keine Partikel eingesetzt. In der Kavität 1 erfolgt die Bindung der Nukleinsäuren an den Plastikkamm mit den aufgesteckten Hülsen. Die gebundene Nukleinsäure wird sukzessive durch die Kavitäten 2 - 4 bewegt. Hier erfolgen die Waschschritte. Danach folgt ein kurzer Trocknungsschritt zur Entfernung von Ethanol. Final wird die Nukleinsäure in der Kavität 5 von den Plastikkämmen mit Hülsen abgelöst. Der Ablauf ist dabei deutlich vereinfacht, da keine Magnetpartikel prozessiert wurden. Das notwendige Sammeln der Magnetpartikel entfällt als Schritt, da ja keine Partikel für die Extraktion eingesetzt wurden. Dadurch war das Verfahren in ca. 15 min beendet. Wie schon aufgeführt, zeigte sich, dass mit allen eingesetzten Plastikmaterialien unter Verwendung einer kommerziell verfügbaren Extraktionschemie und unter Anwendung eines Standard- Extraktionsprotokolls Nukleinsäuren über die Anbindung an das eingesetzte Plastikmaterial isoliert werden können. Diese Beobachtungen waren überraschend. Es war zwar bekannt, dass Biomoleküle unspezifisch an Plastikmaterialien adsorbieren, dies aber aus wässrigen Lösungen, in denen sie vorliegen und dies auch nur in extrem geringen Mengen. Auch gibt es Beschreibungen, Plastikmaterialien für die Isolierung von Nukleinsäuren einzusetzen. Dies aber dadurch, dass man Plastikmaterialien chemisch an ihren Oberflächen so modifiziert, dass sie dieselben funktionalen Gruppen wie silikatische Materialien tragen (OH- Gruppen) oder auch andere funktionale Gruppen an Plastikoberflächen synthetisiert wurden (COOH- Gruppen). Dies ist z.B. in der Patentschrift EP 1135479 aufgeführt. Dem Fachmann ist aber auch bekannt, dass sich solche Mittel zur Isolierung von Nukleinsäuren nicht in der Praxis bewährt haben, da insbesondere die Bindungskapazität viel zu gering ist. Nach diesen ersten Experimenten konnte damit gezeigt werden, dass es möglich ist, mit den gedruckten Plastikhülsen Nukleinsäuren zu isolieren. Allerdings war nicht ersichtlich, warum dies möglich ist. Überraschenderweise konnte die Erklärung mit einem ganz einfachen Experiment gefunden werden. Verwendet wurde wiederum der KingFisher ml. Allerdings wurden bei diesem Versuch keine gedruckten Plastikhülsen eingesetzt. Die Standard-Plastikkämme, welche für die Magnetpartikelprozessierung eingesetzt werden, wurden eingesetzt. Dabei wurden einige Plastikkämme so verwendet, wie sie vorlagen und andere Kämme wurden mittels eines Graviergerätes an der Oberfläche aufgeraut. Es wurde - wie schon beschrieben - eine Extraktion aus Zellen mit denselben beschriebenen Reagenzien und demselben Ablauf durchgeführt. Die detaillierten Ergebnisse sind im Ausführungsbeispiel 1 beschrieben.

Das Experiment zeigt eindrucksvoll, dass an die unbehandelten Kämme keine Nukleinsäure bindet. Im Gegensatz dazu bindet Nukleinsäure hocheffizient an den aufgerauten Kämmen. Damit konnte der Beweis erbracht werden, dass man lediglich die Oberfläche von Plastikmaterialien aufrauen muss, um hocheffizient und unter Verwendung von Standard- Extraktionsreagenzien Nukleinsäuren zu isolieren. Die experimentellen Beobachtungen weisen aber auch noch auf eine andere interessante Tatsache hin. In ersten Versuchen wurden, wie schon beschrieben, Reagenzien von kommerziellen Kits zur Isolierung von Nukleinsäuren eingesetzt. Anstelle der Anbindung der Nukleinsäuren an mineralische feste Phasen (wie z.B. Zentrifugationsfilter-Membranen oder magnetische Partikel) erfolgte die Isolierung der Nukleinsäuren mittels der eingesetzten erfindungsgemäßen rauen Oberflächen. Die eingesetzten Reagenzien waren dabei immer Lysepuffer in Kombination mit einem Bindungspuffer, welcher ein Alkohol war. Es ist bekannt, dass die Lysepuffer Kombinationen aus Salzen, aus Netz-und Dispergiermitteln, Komplexbildner sowie weitere Komponenten sind. Diese Pufferzusammensetzungen in Kombination mit Alkoholen vermitteln die bekannte Anbindung von Nukleinsäuren an mineralische Materialien. Es ist auch schon sehr lange bekannt, dass hohe Konzentrationen chaotroper Salze die Anbindung von Nukleinsäuren an mineralische Materialen vermitteln. Es zeigte sich aber, dass in Anwesenheit einer wässrigen Lösung einer hohen Konzentration eines chaotropen Salzes Nukleinsäuren an ein mineralisches Material binden, nicht aber an eine angeraute Plastikoberfläche. Diese Beobachtung legte den Verdacht nahe, dass der Mechanismus für die Isolierung von Nukleinsäuren mittels rauer Oberflächen ein anderer sein musste, als die bisher bekannten bzw. vermuteten Mechanismen der Anbindung von Nukleinsäuren an mineralische Materialien.

Wie aus dem bereits ausgeführten Stand der Technik bekannt ist, erfolgte unter klassischen Bedingungen die Isolierung von DNA aus Zellen und Geweben dadurch, dass die Nukleinsäuren enthaltenden Ausgangsmaterialien unter stark denaturierenden und reduzierenden Bedingungen, teilweise auch unter Verwendung von proteinabbauenden Enzymen aufgeschlossen, die austretenden Nukleinsäurefraktionen über Phenol-/ Chloroform- Extraktionsschritte gereinigt und die Nukleinsäuren mittels Dialyse oder Ethanolpräzipitation aus der wässrigen Phase gewonnen werden. In diesem Zusammenhang ist dem Fachmann bekannt, dass bei sehr hohen Konzentrationen hochmolekularer DNA nach Ethanolpräzipitation der DNA als "Faden" ausfällt und mittels eines Glasstabes aus dem Reagenzgefäß aufgewickelt werden kann. Dies funktioniert aber nur bei hohen Konzentrationen an DNA und unter der Voraussetzung, dass die DNA auch ein hochmolekulares Molekül ist. Ethanolpräzipitationen basieren deshalb mehrheitlich darauf, dass man nach Alkoholzugabe die Probe zentrifugiert und die DNA als Pellet präzipitiert. Sind die Konzentrationen an DNA gering, dann muss zusätzlich die Probe für einige Stunden bei -20°C inkubiert werden, ehe sie zur Präzipitation der Nukleinsäuren zentrifugiert werden kann. Die Zentrifugationszeit muss in diesen Fällen dann auch stark verlängert werden und kann einige Stunden in Anspruch nehmen. Damit stellt das "aufwickeln" einer hochkonzentrierten und hochmolekularen DNA auf einen Glasstab einen Sonderfall dar. Dieser ist zwar seit Jahrzehnten bekannt, ist aber nicht automatisierbar.

Die zentrifugationsbasierte Präzipitation von Nukleinsäuren dagegen ist zeitaufwendig und ebenfalls schwer zu automatisieren. Aus diesem Grunde Zeit dokumentiert der Stand der Technik ja auch eindrucksvoll, dass die Weiterentwicklung von Technologien zur Isolierung und Aufreinigung von Nukleinsäuren von den klassischen Verfahren hin zur Anbindung von Nukleinsäuren an mineralische Trägermaterialien erfolgte. Umgesetzt werden diese Technologien in der Form, dass man mittels Zentrifugation oder Vakuum eine Nukleinsäure enthaltene Probe an ein Filtermaterial bindet oder mittels magnetischer Separation Nukleinsäuren an magnetische oder paramagnetische Partikel. In Bezug auf die erfindungsgemäße Isolierung von Nukleinsäuren mittels der "Anbindung" an eine raue Oberfläche scheint diese darauf zu basieren, dass die in der Probe enthaltenen Nukleinsäuren nach Inkontaktbringen der Probe mit einer rauen Oberfläche an der rauen Oberfläche präzipitiert. Dies erfolgt dabei dadurch, dass durch die Zugabe z.B. eines Alkohols die Polarität der Umgebung geringer wird und sich dadurch die Löslichkeit der Nukleinsäure reduziert. Überraschenderweise funktioniert die "Präzipitation" der Nukleinsäure an eine raue Oberfläche extrem effizient und erlaubt es somit auch, einfach und schnell und automatisiert Proben mit geringen Konzentrationen an Nukleinsäuren zu isolieren. Es sind dabei keine Zentrifugationsschritte notwendig.

Der Kern der Erfindung besteht somit darin, dass sich freie oder durch Lyse freigesetzte Nukleinsäuren in einer wässrigen Umgebung befinden, deren Polarität mittels organischer Substanzen so eingestellt ist, dass sich die Löslichkeit der Nukleinsäure reduziert und nachfolgend diese wässrige Umgebung mit einer rauen Oberfläche in Kontakt gebracht wird, so dass dann die Nukleinsäure an der rauen Oberfläche präzipitiert und nachfolgend die präzipitierte DNA von der rauen Oberfläche wieder abgelöst wird und zur Verfügung steht. Optional kann die an der rauen Oberfläche präzipitierte Nukleinsäure auch gewaschen und nach Waschschritten abgelöst werden.

Die vorliegende Erfindung ermöglicht damit in idealster Weise die Umsetzung der schon aufgeführten Zielstellung. Für die Extraktion benötigt man lediglich ein Material, dessen Oberfläche aufgeraut ist und eine wässrige Umgebung in der sich die zu isolierende Nukleinsäure befindet, deren Bedingungen mittels einer organischen Substanz so eingestellt sind, dass sich die Löslichkeit der Nukleinsäure so verringert, dass diese an der rauen Oberfläche präzipitiert. Diese Präzipitation an der rauen Oberfläche erfolgt durch Inkontaktbringen der rauen Oberfläche mit der Probe oder durch Inkontaktbringen der Probe mit der rauen Oberfläche. Als Extraktionsreagenzien können z.B. klassische Kitreagenzien (z.B. Lysepuffer) unterschiedlicher Art verwendet werden. Die notwendigen Bedingungen für die Präzipitation der Nukleinsäure an die raue Oberfläche werden durch die Zugabe einer organischen Substanz eingestellt. Ebenso arbeitet das Extraktionsprotokoll nach dem bekanntem Schema Lysieren- Binden- Waschen- Eluieren. Alternativ dazu können Waschschritte auch wegelassen werden. Das Verfahren ist aber nunmehr extrem einfach und schnell in der Durchführung. Es kann in folgenden Schritten durchgeführt werden:
1. Lysieren einer Nukleinsäure enthaltenden Probe (oder Bereitstellen einer Nukleinsäure enthaltenden flüssigen Probe). Für die Lyse einer Probe wird die Probe mit einem klassischen Lysepuffer oder einem Puffer versetzt, die man bisher aus Verfahren zur Isolierung von Nukleinsäuren kennt. Diese Puffer können chaotrope Salze oder nichtchaotrope Salze oder Mischungen dieser beiden Gruppen enthalten. Darüber hinaus können diese Puffer weitere Komponenten enthalten wie Chealtbildner, Trispuffer, Netz-und Dispergiermittel etc. Das Verfahren funktioniert aber auch mit Puffern, welche keine Salze enthalten und z.B. nur aus einem Detergenz, Tris und EDTA bestehen. Zusätzlich können auch noch proteolytische Enzyme eingesetzt werden.
2. Zugabe eines Bindungspuffers, welcher eine alkoholische Komponente und weitere Zusätze enthält oder Zugabe eines Alkohols allein oder auch Zugabe von Aceton oder Benzin.
3. Inkontaktbringen dieser Mischung mit einem Material dessen Oberfläche rau ist, wobei die Rauheit der Oberfläche durch Berühren oder durch Ansicht der Oberfläche erkennbar is.
4. Entfernen des rauen oder eine Struktur aufweisenden Materials mit der angebundenen Nukleinsäure aus der Mischung oder Entfernen der Mischung vom Material.
5. Ggf. Waschen des Materials.
6. Ggf. Trocknung des Materials.
7. Ablösen der Nukleinsäure vom Material mit einem Elutionspuffer (Niedrigsalzpuffer oder Wasser).

Das Verfahren ist universell einsetzbar und kann sowohl automatisiert als auch manuell durchgeführt werden. Damit ist es auch in idealster Weise für den Einsatz einer Nukleinsäure-Extraktion unter Feldbedingungen nutzbar, da die notwendigen Schritte einfach durchführbar sind. Die einzusetzenden erfindungsgemäßen Materialien sind ebenfalls nicht limitierend.

Eingesetzt werden können auch sogenannte Kompositmaterialien, die Mischungen aus Polymeren und z.B. organischen Komponenten oder metallischen Komponenten darstellen. Wesentlich ist nur die Bereitstellung einer angerauten Oberfläche. Die Architektur des erfindungsgemäßen Materials ist ebenfalls nicht limitierend. Vorteilhaft ist auch die Verwendung von rauem, magnetischen Material. Solch ein Material ist als Granulat unter dem Markennamen TECACOPM^{®} bekannt. Beispielsweise kann auch eine Standardpipettenspitze an ihrer Innenseite gemäß der Erfindung aufgeraut werden und für die Extraktion von Nukleinsäuren manuell oder automatisiert eingesetzt werden. Dazu wird entsprechend des beschriebenen Extraktions-workflows mittels multipler Pipettierschritte das Extraktionsprotokoll sukzessive abgearbeitet. Dem Fachmann wird damit deutlich, wie extrem einfach mittels der vorliegenden Erfindung nunmehr eine Extraktion von Nukleinsäuren durchgeführt werden kann. Die vorliegende Erfindung zeigt einen weiteren enormen Vorteil. Enthält eine biologische Probe große Mengen an Nukleinsäuren, so kann mittels des erfindungsgemäßen Verfahrens und der erfindungsgemäßen Plastik eine extrem große Menge an Nukleinsäuren extrahiert werden. Dies Ausbeuten übersteigen dabei die erzielbaren Ausbeuten mit bekannten Extraktionsverfahren unter Verwendung von mineralischen Trägermaterialien um ein Vielfaches. Damit ist das Verfahren auch ideal für die Bearbeitung von großen Probenvolumina geeignet. Auch zeigt sich, dass sowohl genomische DNA, RNA als auch Plasmid DNA isoliert werden können. Das erfindungsgemäße Verfahren kann als automatisierter Extraktionsprozess multiple Proben extrem schnell bearbeiten. Beispielhaft können mittels des erfindungsgemäßen Verfahrens unter der zweckentfremdeten Verwendung des KingFisher ml, 15 Proben in 15 Minuten gleichzeitig bearbeitet werden. Auch kann dieses Verfahren ohne Verlust an Quantität und Qualität der zu isolierenden Nukleinsäure weiter verkürzt werden. Dies kann dann auch auf andere Automatenstationen übertragen werden. Z.B. kann die Extraktion auch in aufgerauten Pipettenspitzen mit allen gängigen Pipettierautomaten umgesetzt werden. Mit der Erfindung steht damit eine völlig neuartige Plattformtechnologie für die Isolierung und Aufreinigung von Nukleinsäuren zur Verfügung, welche eine Vielzahl von ganz deutlichen Vorteilen gegenüber den bekannten Extraktionsverfahren zeigt. Gegenstand der Erfindung ist somit auch ein Gerät zur Isolierung von Nukleinsäuren, umfassend angeraute Pipettenspitzen.

Die Erfindung soll nachfolgend anhand von Ausführungsbeispielen näher erläutert werden. Die Ausführungsbeispiele stellen dabei keine Limitierung der Erfindung dar.

### Ausführungsbeispiele

### Beispiel 1. Nachweis der Bindung von Nukleinsäuren an rauen Oberflächen unter Nutzung einer kommerziell verfügbaren Extraktionschemie

Der Nachweis, dass Nukleinsäuren an raue Oberflächen binden und isoliert werden können, wurde wie folgt durchgeführt. Die Extraktion wurde dabei automatisiert umgesetzt. Zum Einsatz kam ein Magnetpartikelprozessor (KingFisher ml; Thermo Electron). Das Gerät verwendet Plastikkämme, in welche Magnetstäbe einfahren, welche dann in einem walk-away- Prozess Magnetpartikel bewegen, die für die Isolierung von Nukleinsäuren eingesetzt werden. Diese Plastikkämme wurden für das erfindungsgemäße Verfahren zweckentfremdet eingesetzt und sollten der Bindung und nachfolgenden Extraktion der Nukleinsäure dienen. Das Plastikmaterial ist Polypropylen. Gemäß der vorliegenden Erfindung wurden die Plastikkämme mittels eines Schleifgerätes angeraut. Eingesetzt wurden ebenfalls unbehandelte Plastikkämme. Als Probe wurden jeweils ca. 1 × 10⁶NIH 3T3 Zellen verwendet. Die für die Isolierung der Nukleinsäuren eingesetzte Extraktionschemie wurde dabei teilweise aus dem kommerziellen Extraktionskit innuPREP Blood DNA Kit/IPC16X (Analytik Jena AG) genommen. Mittels eines Lysepuffers (Lysis Solution CBV) sowie von Proteinase K wurden die Zellen bei 60°C für 15 min lysiert. Während der Lyse wurde die Reaktionsplastik des KingFisher ml mit folgenden Lösungen befüllt:
Kavität 1: 400 µl Isopropanol
Kavität 2: 800 µl Washing Solution LS (aus dem Extraktionskit)
Kavität 3: 800 µl 80% Ethanol
Kavität 4: 800 µl 80% Ethanol
Kavität 5: 200 µl Elution Buffer (aus dem Extraktionskit)

Nach erfolgter Lyse wurde das Lysat (400 µl) in die Kavität 1 zum dort schon vorliegenden Isopropanol gegeben und der Extraktionsprozess gestartet. Bei diesem Prozess bewegen sich die Plastikkämme sukzessive von Kavität 1 bis Kavität 5. Dabei erfolgt in jeder Kavität eine vertikale Bewegung der Plastikkämme innerhalb der jeweils vorliegenden Pufferlösung. Der Extraktionsprozess basiert dabei auf den klassischen Prinzipien der Nukleinsäureisolierung unter Verwendung von magnetischen Partikeln. Es werden aber keine Partikel eingesetzt. In der Kavität 1 erfolgt die Bindung der Nukleinsäuren an den Plastikkamm. Die gebundene Nukleinsäure wird sukzessive durch die Kavitäten 2 - 4 bewegt. Hier erfolgen die Waschschritte. Danach folgt ein kurzer Trocknungsschritt zur Entfernung von Ethanol. Final wird die Nukleinsäure in der Kavität 5 von den Plastikkämmen abgelöst. Wie schon aufgeführt, wurden dabei Plastikkämme eingesetzt, deren Oberfläche aufgeraut wurden sowie die ursprünglichen unbehandelten Plastikkämme. Der Nachweis der isolierten Nukleinsäure erfolgte mittels spektrophotometrischer Messung sowie auf einem Agarosegel. Neben der Ausbeute wurde auch die Reinheit der isolierten Nukleinsäure bestimmt.

Ergebnisse der spektrophotometrischen Vermessung:

| Probe | Konzentration (ng/ µl) | Ausbeute (µg) | Ratio A₂₆₀:A₂₈₀ | Ratio A₂₆₀:A₂₃₀ |
|---|---|---|---|---|
| unbehandelter Plastikkamm 1 | 0 | nicht messbar | nicht messbar | nicht messbar |
| unbehandelter Plastikkamm 2 | 0 | nicht messbar | nicht messbar | nicht messbar |
| aufgerauter Plastikkamm 1 | 56 | 11,2 | 1,90 | 2,46 |
| aufgerauter Plastikkamm 2 | 60 | 12 | 1,86 | 2,30 |
| aufgerauter Plastikkamm 3 | 63 | 12,6 | 1,96 | 2,39 |

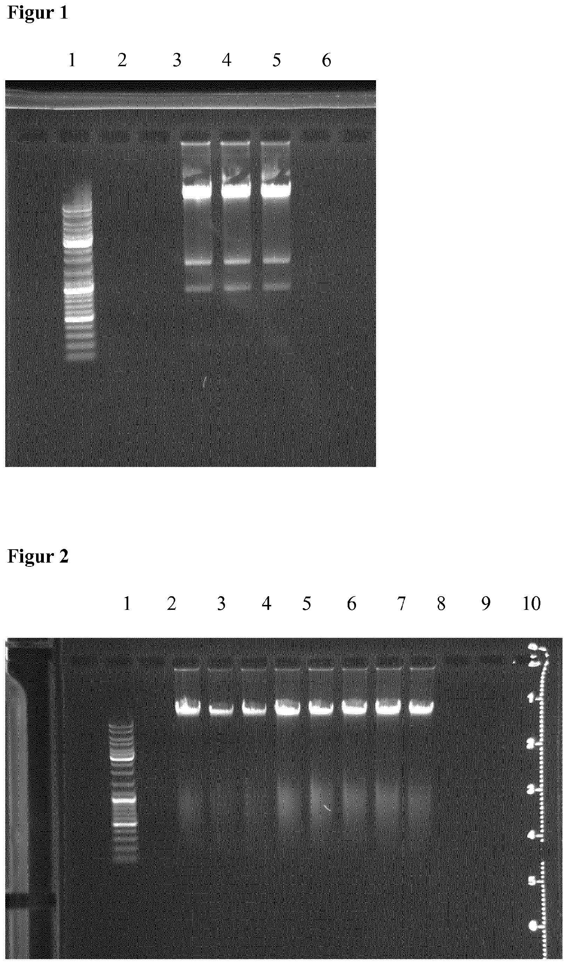

Figur 1 zeigt den Nachweis der DNA auf einem Agarosegel. Je Spur wurden 4 µl Nukleinsäure vom Gesamteluat von 200 µl eingesetzt. Die Spuren bedeuten:
1. DNA Leiter (1b Leiter)
2. unbehandelter Plastikkamm
3. unbehandelter Plastikkamm
4. aufgerauter Plastikkamm 1
5. aufgerauter Plastikkamm 2
6. Aufgerauter Plastikkamm 3

Wie die Ergebnisse eindrucksvoll zeigen, bindet die Nukleinsäure (sowohl DNA als auch RNA) an den unbehandelten Kämmen nicht. Sie bindet aber hocheffizient an den aufgerauten Kämmen und kann nachfolgend mit klassischen Waschpuffern gewaschen und final wieder von den Kämmen abgelöst werden. Damit entspricht der Prozess exakt den klassischen Abläufen der Isolierung von Nukleinsäuren mittels magnetischer Partikel oder mittels dem Fachmann bekannter mineralischer Trägermaterialien. Der Prozess ist aber sehr viel schneller und einfacher in der Durchführung. Mit diesem Beispiel wurde damit gezeigt, dass allein durch ein Anrauen einer Plastikoberfläche dieses Material für die Extraktion von Nukleinsäuren unter Verwendung bekannter Extraktionsreagenzien eingesetzt werden kann.

### Beispiel 2: Testung unterschiedlicher Plastikmaterialien mit rauen Oberflächen für die Extraktion von Nukleinsäuren unter Nutzung einer kommerziell verfügbaren Extraktionschemie

Der Nachweis, dass Nukleinsäuren an raue Oberflächen von unterschiedlichen Plastikmaterialien binden und isoliert werden können, wurde wie folgt durchgeführt. Die Extraktion wurde dabei automatisiert umgesetzt. Zum Einsatz kam wieder der Magnetpartikelprozessor KingFisher ml. Die Plastikkämme wurden für das erfindungsgemäße Verfahren wieder zweckentfremdet eingesetzt. Als Material zur Bindung der Nukleinsäure diente wie im Ausführungsbeispiel 1 ein angerauter Plastikkamm. Als Kontrolle wurde ein nicht behandelter Kamm eingesetzt. Zur Testung anderer Materialien wurden mittels eines 3-D- Druckers Plastikringe aus unterschiedlichen Materialien hergestellt, welche wiederum auf die Kämme des KingFisher ml Gerätes gesteckt wurden. Auch diese Ringe wurden wieder mit einem Schleifgerät angeraut. Bei den unterschiedlichen Materialien handelte es sich um angeraute Kämme der Plastik des KingFisher ml (Polypropylen), des Weiteren um das Material Biofila Linen (Fa. TwoBEars), einem Kompositmaterial bestehend aus Lignin und einem Komplexpolymer aus aromatischen Alkoholen, um das Material Acrylnitril- Butadien-Styrol (ABS), das Material aus Polylaktit (PLA), um das Material Polycarbonat (PC) sowie das Material Polystyrol (PS). Die Kämme vom KingFisher ml wurden dabei unterschiedlich angeraut (Typ A: ca. 3 cm des Kammes, Typ B: ca. 1 cm des Kammes; C. nur die Spitze des Kammes). Als Probe wurden jeweils ca. 1 × 10⁶NIH 3T3 Zellen verwendet. Die für die Isolierung der Nukleinsäuren eingesetzte Extraktionschemie wurde wiederum teilwiese aus dem kommerziellen Extraktionskit innuPREP Blood DANN Kit/IPC16 (Analytik Jena AG) genommen. Unter Verwendung eines Lysepuffers (Lysis Solution CBV) sowie von Proteinase K wurden die Zellen bei 60°C für 15 min lysiert. Während der Lyse wurde die

Reaktionsplastik des KingFisher ml mit folgenden Lösungen befüllt:
Kavität 1: 400 µl Isopropanol
Kavität 2: 800 µl Washing Solution LS (aus dem Extraktionskit)
Kavität 3: 800 µl 80% Ethanol
Kavität 4: 800 µl 80% Ethanol
Kavität 5: 200 µl Elution Buffer (aus dem Extraktionskit)

Nach erfolgter Lyse wurde das Lysat (400 µl) in die Kavität 1 zum dort schon vorliegenden Isopropanol gegeben und der Extraktionsprozess gestartet. Der Ablauf der Extraktion erfolgte wie im Ausführungsbeispiel 1 beschrieben. Die Plastikkämme (unbehandelt bzw. angeraut) sowie die Plastikkämme mit den aufgesteckten Plastikringen wurden wiederum sukzessive durch die einzelnen Kavitäten mit den vorgelegten Pufferlösungen bewegt. Final wurde die Nukleinsäure in der Kavität 5 von den Plastikkämmen abgelöst. Der Nachweis der isolierten Nukleinsäure erfolgte mittels spektrophotometrischer Messung sowie auf einem Agarosegel. Neben der Konzentration sowie Ausbeute wurde auch die Reinheit der isolierten Nukleinsäure bestimmt.

Ergebnisse der spektrophotometrischen Vermessung:

| Probe | Konzentration (ng/ µl) | Ausbeute (µg) | Ratio A₂₆₀:A₂₈₀ | Ratio A₂₆₀:A₂₃₀ |
|---|---|---|---|---|
| unbehandelter Plastikkamm | keine Nukleinsäure messbar | nicht bestimmbar | nicht bestimmbar | nicht bestimmbar |
| aufgerauter Plastikkamm Typ A | 84 | 16,8 | 1,91 | 1,98 |
| aufgerauter Plastikkamm Typ B | 95 | 19 | 1,92 | 2,01 |
| aufgerauter Plastikkamm Typ C | 75 | 15 | 1,90 | 2,11 |
| Plastikkamm mit aufgerautem Ring aus BioFila | 178 | 35,6 | 1,95 | 2,22 |
| Plastikkamm mit aufgerautem Ring aus ABS | 179 | 35,8 | 2,03 | 2,30 |
| Plastikkamm mit aufgerautem Ring aus PLA | 205 | 41 | 1,94 | 1,98 |
| Plastikkamm mit aufgerautem Ring aus PC | 185 | 37 | 1,99 | 2,28 |
| Plastikkamm mit aufgerautem Ring aus PS | 157 | 31,4 | 1,96 | 2,32 |

Figur 2 zeigt den Nachweis der DNA auf einem Agarosegel. Je Spur wurden 4 µl Nukleinsäure vom Gesamteluat von 200 µl eingesetzt. Die Spuren zeigen:
1. DNA Leiter (1b Leiter)
2. unbehandelter Plastikkamm
3. aufgerauter Plastikkamm Typ A
4. aufgerauter Plastikkamm Typ B
5. aufgerauter Plastikkamm Typ C
6. Plastikkamm mit aufgerautem Ring aus BioFila
7. Plastikkamm mit aufgerautem Ring aus ABS
8. Plastikkamm mit aufgerautem Ring aus PLA
9. Plastikkamm mit aufgerautem Ring aus PC
10. Plastikkamm mit aufgerautem Ring aus PS

Wie die Ergebnisse zeigen, bindet die Nukleinsäure an den unbehandelten Kämmen nicht. Sie bindet aber hocheffizient an den aufgerauten Kämmen und den aufgerauten Ringen. Damit ist gezeigt, dass die Bindung unabhängig von der Materialart ist. Alle angerauten Materialien binden hocheffizient Nukleinsäuren. Es zeigt sich ebenfalls, dass die angeraute Fläche (im Falle der Kämme) sogar nur sehr gering sein muss, um Nukleinsäuren zu binden. Es zeigt sich aber auch, dass größere Flächen auch noch höhere Mengen an Nukleinsäuren binden (die Flächen bei den eingesetzten Ringen ist größer als die angerauten Oberflächen der Kämme). Die Qualität der Nukleinsäuren ist exzellent.

### Beispiel 3: Vergleich eines klassischen Kits zur Isolierung von Nukleinsäuren mit dem erfindungsgemäßen Verfahren

Mit diesem Beispiel sollte der Vergleich zwischen einem klassischen Kit zur Isolierung von Nukleinsäuren mit dem erfindungsgemäßen Verfahren durchgeführt werden. Als Vergleichs Kit wurde der Kit DNeasy Blood&Tissue Kit der Fa. Qiagen eingesetzt. Dieser basiert auf der Lyse der Zellen, der Bindung der Nukleinsäuren an die Oberfläche einer Spin Filtersäule mit einer Silikamembran, dem nachfolgenden Waschen der gebundenen Nukleinsäure und der finalen Elution der Nukleinsäure von der Silikamembran. Dieses Kit ist ein Standard Kit zur Extraktion von Nukleinsäuren und wird weltweit dafür eingesetzt. Es wurde nach vorliegendem Manual gearbeitet.

Für das erfindungsgemäße Verfahren wurde wiederum der KingFisher ml eingesetzt. Als raue Oberfläche wurden ein aufgerauter Kamm sowie ein auf den Kamm gesteckter Ring aus angerautem PLA eingesetzt. Der Ablauf der Extraktion mittels des erfindungsgemäßen Verfahrens erfolgte wie in den beiden Ausführungsbeispielen 1 und 2 aufgeführt. Als Probe wurden jeweils ca. 1 × 10⁶NIH 3T3 bzw. 2 × 10⁶ NIH 3T3 Zellen verwendet. Der Nachweis der isolierten Nukleinsäure erfolgte mittels spektrophotometrischer Messung sowie auf einem Agarosegel. Neben der Konzentration sowie Ausbeute wurde auch die Reinheit der isolierten Nukleinsäure bestimmt.

Ergebnisse der spektrophotometrischen Vermessung:

| Probe | Konzentration (ng/ µl) | Ausbeute (µg) | Ratio A₂₆₀:A₂₈₀ | Ratio A₂₆₀:A₂₃₀ |
|---|---|---|---|---|
| Qiagen Kit (1 × 10⁶ Zellen) | 49 | 9,8 | 1,88 | 2,15 |
| Qiagen Kit (2 × 10⁶ Zellen) | 57 | 11,4 | 1,84 | 2,21 |
| aufgerauter Plastikkamm (1 × 10⁶ Zellen) | 101 | 20,2 | 1,87 | 2,04 |
| aufgerauter Plastikkamm (2 × 10⁶ Zellen) | 147 | 29,4 | 1,98 | 2,15 |
| Plastikkamm mit aufgerautem Ring aus PLA (1 × 10⁶ Zellen) | 240 | 48 | 1,96 | 2,20 |
| Plastikkamm mit aufgerautem Ring aus PLA (2 × 10⁶ Zellen) | 503 | 100,6 | 1,96 | 2,20 |

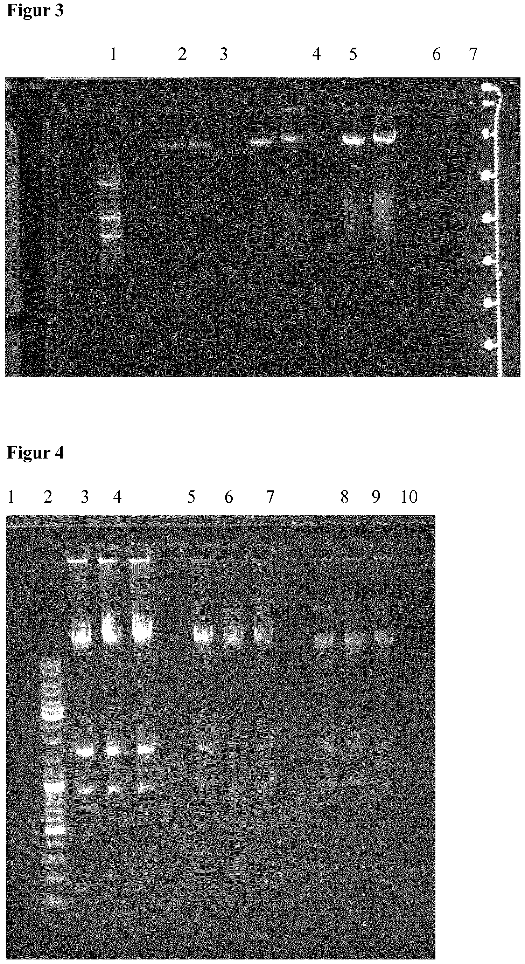

Figur 3 zeigt den Nachweis der DNA auf einem Agarosegel. Je Spur wurden 2 µl Nukleinsäure vom Gesamteluat von 200 µl eingesetzt. Die Spuren zeigen:
1. DNA Leiter (1b Leiter)
2. Qiagen Kit (ca. 1 × 10⁶ Zellen)
3. Qiagen Kit (ca. 2 × 10⁶ Zellen)
4. erfindungsgemäßes Verfahren; aufgerauter Plastikkamm (ca. 1 × 10⁶ Zellen)
5. erfindungsgemäßes Verfahren; aufgerauter Plastikkamm (ca. 2 × 10⁶ Zellen)
6. erfindungsgemäßes Verfahren; Plastikkamm mit aufgerautem Ring aus PLA (ca. 1 × 10⁶ Zellen)
7. erfindungsgemäßes Verfahren; Plastikkamm mit aufgerautem Ring aus PLA (ca. 2 × 10⁶ Zellen)

Wie die Ergebnisse zeigen, kann mit dem erfindungsgemäßen Verfahren eine sehr viel höhere Menge an Nukleinsäuren gebunden und isoliert werden, als mit einem klassischen Kit. Damit sind die Bindungskapazitäten deutlich höher, als die Bindungskapazitäten eines kommerziell verfügbaren Silikamembran-Verfahrens. Dies unterstreicht den enormen Vorteil des erfindungsgemäßen Verfahrens.

### Beispiel 4: Extraktion von Nukleinsäure aus Blut

Mit diesem Beispiel wird gezeigt, dass mittels des erfindungsgemäßen Verfahrens auch aus Blutproben DNA isoliert werden kann und dabei die Ausbeuten extrem hoch sind. Eingesetzt wurden 3 ml Vollblutproben. Nach Lyse der Erythrozyten wurden die kernhaltigen Zellen pelletiert und wiederum nach Zugabe eines Lysepuffers (Lysepuffer CBV) aus dem kommerziell verfügbaren Kit innuPREP Blood DNA Kit/IPC16 (Analytik Jena AG) sowie unter Zugabe von Proteinase K bei 60°C für 30 min lysiert. Eluiert wurde in 300 µl Elution Buffer.

Für das erfindungsgemäße Verfahren wurde wiederum der KingFisher ml eingesetzt. Als raue Oberflächen wurde ein auf den Kamm gesteckter Ring aus angerautem Material BioFila sowie PLA eingesetzt. Der Ablauf der Extraktion mittels des erfindungsgemäßen Verfahrens erfolgte wie in den Ausführungsbeispielen 1 bis 3 aufgeführt.

Der Nachweis der isolierten Nukleinsäure erfolgte mittels spektrophotometrischer Messung.

Ergebnisse der spektrophotometrischen Vermessung:

| Probe | Konzentration (ng/ µl) | Ausbeute (µg) | Ratio A₂₆₀:A₂₈₀ | Ratio A₂₆₀:A₂₃₀ |
|---|---|---|---|---|
| Plastikkamm mit aufgerautem Ring aus Biofila | 270 | 81 | 1,78 | 2,22 |
| Plastikkamm mit aufgerautem Ring aus PLA | 306 | 91,8 | 1,80 | 2,22 |

Wie die Ergebnisse zeigen, ist es mit dem erfindungsgemäßen Verfahren möglich, Nukleinsäuren auch aus Vollblutproben zu isolieren, wobei die erzielbaren Ausbeuten extrem hoch sind.

### Beispiel 5: Extraktion von Nukleinsäure aus NIH 3T3 Zellen sowie aus Vollblutproben mittels des erfindungsgemäßen Verfahrens unter Verwendung einer modifizierten Pipettenspitze

Mit diesem Beispiel wird gezeigt, dass mittels des erfindungsgemäßen Verfahrens extrem einfach und schnell Nukleinsäuren mittels einer modifizierten Pipettenspitze isoliert werden können.

Als Pipettenspitze wurde eine 1ml Pipettenspitze der Fa. Sarstedt eingesetzt. Diese Pipettenspitze wurde um ca. 5 mm gekürzt. Entsprechend dem erfindungsgemäßen Verfahren wurde dann die Innenseite der Pipettenspitze mittels eines Schleifgerätes aufgeraut.

Eingesetzt wurden 1 × 10⁶ NIH 3T3 Zellen sowie 2 ml Vollblut (die Erythrozyten wurden lysiert und die kernhaltigen Zellen pelletiert und diese Zellen dann eingesetzt). Sowohl die Zellen als auch die pelletierten kernhaltigen Blutzellen wurden wie in den vorangegangenen Ausführungsbeispielen behandelt und mit dem Lysepuffer CBV lysiert. Das Lysat wurde in ein 2 ml Reaktionsgefäß überführt und mit 400 µl Isopropanol versetzt. Nachfolgend wurde die aufgeraute Pipettenspitze eingesetzt und mittels einer Pipette wurde der Ansatz 20 × auf- und abpipettiert. Danach wurden 3 weitere 2 ml- Reaktionsgefäße mit den bekannten alkoholischen Waschpuffern (LS, 80%iger Ethanol, 80%iger Ethanol) befüllt. Die Pipettenspitze wurde dann sukzessive in die jeweiligen Waschpuffer getaucht und es wurde jeweils 5 × auf- und abpipettiert. Nach dem letzten Waschschritt wurde die Spitze getrocknet und damit der restliche Ethanol entfernt. Die Elution der gebundenen Nukleinsäure erfolgte mit 200 µl Elution Buffer für die NIH 3T3 Zellen sowie mit 400 µl für die Vollblutprobe. Dieser wurde wiederum in ein 2 ml- Reaktionsgefäß gegeben. Es wurde 20 × auf- und abpipettiert. Nach Entfernung der Pipettenspitze liegt die isolierte Nukleinsäure im Reaktionsgefäß vor. Das Verfahren ist extrem einfach und schnell.

Der Nachweis der isolierten Nukleinsäure erfolgte mittels spektrophotometrischer Messung.

Ergebnisse der spektrophotometrischen Vermessung:

| Probe | Konzentration (ng/ µl) | Ausbeute (µg) | Ratio A₂₆₀:A₂₈₀ | Ratio A₂₆₀:A₂₃₀ |
|---|---|---|---|---|
| ca. 1 × 10⁶ NIH 3T3 Zellen; unbehandelte Pipettenspitze | keine Nukleinsäure messbar | nicht bestimmbar | nicht bestimmbar | nicht bestimmbar |
| ca. 1 × 10⁶ NIH 3T3 Zellen; angeraute Pipettenspitze | 84 | 16,8 | 1,97 | 1,88 |
| 2 ml Vollblut (Lymphozytenpellet); unbehandelte Pipettenspitze | keine Nukleinsäure messbar | nicht bestimmbar | nicht bestimmbar | nicht bestimmbar |
| 2ml Vollblut (Lymphozytenpellet); angeraute Pipettenspitze | 235 | 94,0 | 1,79 | 2,17 |

Wie die Ergebnisse zeigen, ist es mit dem erfindungsgemäßen Verfahren möglich, allein unter Verwendung einer Pipettenspitze, deren Innenseite erfindungsgemäß aufgeraut wurde, Nukleinsäure zu Binden und zu isolieren. Auch hier zeigt sich, dass die Ausbeuten extrem hoch sind. Mittels der unbehandelten Pipettenspitze können keine Nukleinsäuren isoliert werden.

### Beispiel 6: Testung unterschiedlicher Lysepuffer in Kombination mit unterschiedlichen alkoholischen bzw. nichtalkoholischen Lösungen zur Extraktion von Nukleinsäuren mittels des erfindungsgemäßen Verfahrens

Eingesetzt wurde wiederum der Magnetpartikelprozessor KingFisher ml. Als Material zur Bindung der Nukleinsäure diente ein mit einem Schleifgerät angerauter Ring aus BioFila Material, welcher auf die Kämme der Plastik des KingFisher ml aufgesteckt wurden. Als Probe wurden jeweils ca. 1 × 10⁶NIH 3T3 Zellen verwendet. Die Lyse der Zellen wurde mit drei verschiedenen Puffern durchgeführt:
1. Puffer A, enthaltend ein chaotropes Salz: Harnstoff, SDS, Tris HCl, EDTA
2. Puffer B, enthaltend kein Salz: SDS; Tris HCl; EDTA
3. Puffer C enthaltend ein nichtchaotropes Salz: Natriumchlorid, CTAB; Tris HCl, EDTA

Die Zellen wurden in 200 µl H₂O resuspendiert und mit 200 µl des jeweiligen Lysepuffers sowie 20 µl versetzt und bei 60°C für 15 min lysiert. Während der Lyse wurde die Reaktionsplastik des KingFisher ml mit folgenden Lösungen befüllt:
Kavität 1: 400 µl Isopropanol oder absoluter Ethanol oder Aceton
Kavität 2: 800 µl Washing Solution LS (aus dem Extraktionskit innuPREP Blood DNA Kit/IPC16)
Kavität 3: 800 µl 80% Ethanol
Kavität 4: 800 µl 80% Ethanol
Kavität 5: 200 µl Elution Buffer (aus dem Extraktionskit innuPREP Blood DNA Kit/IPC16)

Nach erfolgter Lyse wurde das Lysat (400 µl) in die Kavität 1 zu dort schon vorliegenden verschiedenen Lösungen (Isopropanol; absoluter Ethanol bzw. Aceton) gegeben und der Extraktionsprozess gestartet. Der Ablauf der Extraktion erfolgte wie im Ausführungsbeispiel 1 beschrieben. Der Nachweis der isolierten Nukleinsäure erfolgte mittels spektrophotometrischer Messung sowie auf einem Agarosegel. Neben Konzentration und Ausbeute wurde auch die Reinheit der isolierten Nukleinsäure bestimmt.

Ergebnisse der spektrophotometrischen Vermessung:

| Probe | Konzentration (ng/ µl) | Ausbeute (µg) | Ratio A₂₆₀:A₂₈₀ | Ratio A₂₆₀:A₂₃₀ |
|---|---|---|---|---|
| Lysepuffer A + Isopropanol | 174 | 34,8 | 1,90 | 2,10 |
| Lysepuffer A + abs. Ethanol | 213 | 42,6 | 1,89 | 2,11 |
| Lysepuffer A + Aceton | 220 | 44 | 1,92 | 2,17 |
| Lysepuffer B + Isopropanol | 150 | 30 | 1,90 | 2,11 |
| Lysepuffer B + abs. Ethanol | 106 | 21,2 | 1,80 | 1,99 |
| Lysepuffer B + Aceton | 128 | 25,6 | 1,87 | 1,97 |
| Lysepuffer C + Isopropanol | 112 | 22,4 | 1,85 | 1,97 |
| Lysepuffer C + abs. Ethanol | 98 | 19,6 | 1,86 | 2,00 |
| Lysepuffer C + Aceton | 89 | 17,8 | 1,87 | 1,99 |

Figur 4 zeigt den Nachweis der DNA auf einem Agarosegel. Je Spur wurden 4 µl Nukleinsäure vom Gesamteluat von 200 µl eingesetzt. Die Spuren zeigen:
1. DNA Leiter (1b Leiter)
2. Lysepuffer A + Isopropanol
3. Lysepuffer A + abs. Ethanol
4. Lysepuffer A + Aceton
5. Lysepuffer B + Isopropanol
6. Lysepuffer B + abs. Ethanol
7. Lysepuffer B + Aceton
8. Lysepuffer C + Isopropanol
9. Lysepuffer C + abs. Ethanol
10. Lysepuffer C + Aceton

Wie die Ergebnisse zeigen, können für das erfindungsgemäße Verfahren unterschiedlich zusammengesetzte Lysepuffer wie auch Kombination dieser Lysepuffer mit Alkoholen bzw. auch mit einem Nichtalkohol eingesetzt werden. Dabei können im Lysepuffer chaotrope Salze, kein Salz oder aber nichtchaotrope Salze enthalten sein.

### Beispiel 7. Nachweis der Rückgewinnung genomischer DNA aus einer wässrigen Lösung

Es wurde eine wässrige Lösung hergestellt, welche genomische DNA, die aus Blut isoliert wurde, enthielt. 300 µl dieser DNA Lösung wurde mit 30 µl einer 3 molaren Natriumacetat Lösung versetzt sowie mit 300 µl Isopropanol.

Der Nachweis, dass auch bereits schon isolierte Nukleinsäuren an raue Oberflächen binden und isoliert werden können, wurde wie folgt durchgeführt. Die Extraktion wurde dabei automatisiert umgesetzt. Für das erfindungsgemäße Verfahren wurde wiederum der KingFisher ml eingesetzt. Als raue Oberfläche wurden ein auf den Kamm gesteckter Ring aus Biofila - Material eingesetzt. Dieser Ring wurde mittels 3-D-Drucktechnik hergestellt und enthielt auf der Oberfläche eine Rillenstruktur.

Die Reaktionsplastik des KingFisher ml wurde mit folgenden Lösungen befüllt:
Kavität 1: DNA Lösung /Natriumacetat/ Isopropanol
Kavität 2: 800 µl 80 % Ethanol
Kavität 3: leer
Kavität 4: leer
Kavität 5: 150 µl Wasser

Der Ablauf der Extraktion erfolgte wie im Ausführungsbeispiel 1 beschrieben, wobei diesmal nur ein Waschschritt durchgeführt wurde. Der Nachweis der isolierten Nukleinsäure erfolgte auf einem Agarosegel.

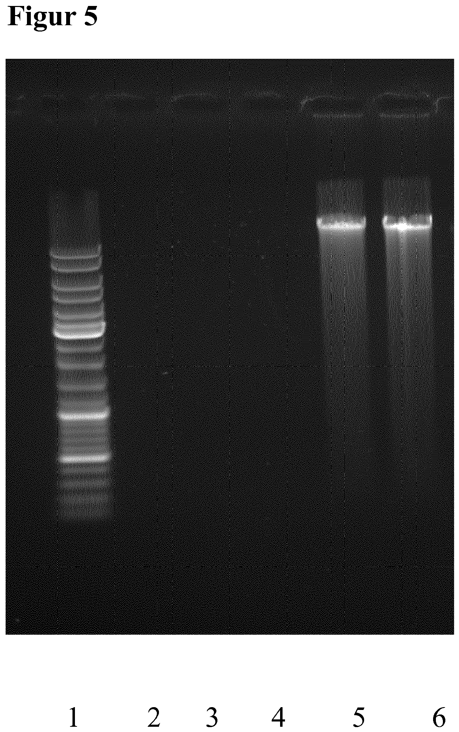

Figur 5 zeigt den Nachweis der DNA auf einem Agarosegel. Je Spur wurden 10 µl Nukleinsäure vom Gesamteluat von 150 µl eingesetzt. Die Spuren bedeuten:
1. DNA Leiter (1b Leiter)
2. leer
3. unbehandelter Plastikkamm
4. unbehandelter Plastikkamm
5. Plastikkamm mit aufgestecktem Ring aus Biofila und Rillenstruktur
6. Plastikkamm mit aufgestecktem Ring aus Biofila und Rillenstruktur

Wie die Ergebnisse eindrucksvoll zeigen, bindet die Nukleinsäure an den unbehandelten Kämmen nicht. Sie bindet aber an den Ringen, welche eine Rillenstruktur besitzen. Damit wurde bewiesen, dass man auch eine bereits vorliegende DNA aus einer wässrigen Lösung zurückgewinnen kann. Dur die Zugabe von Isopropanol und Natriumacetat wurde die Löslichkeit der DNA herabgesetzt, so dass die an die Oberfläche des Plastikmaterial binden konnte.

## Patentansprüche

1. Verfahren zur Isolierung von Nukleinsäuren aus wässrigen, Nukleinsäure-enthaltenden Proben, **dadurch gekennzeichnet, dass** eine wässrige Lösung, die freie oder durch Lyse freigesetzte Nukleinsäuren enthält, vor oder nach Senkung der Polarität der wässrigen Lösung mit einer festen Phase, die eine raue Oberfläche aus Kunststoff aufweist, in Kontakt gebracht wird, wobei die Rauheit der Oberfläche durch Berühren oder durch Ansicht der Oberfläche erkennbar ist und die Nukleinsäuren an der festen Phase präzipitieren und anschließend aus dieser wässrigen Lösung mit der festen Phase entfernt werden.

2. Verfahren nach Anspruch 1, **dadurch gekennzeichnet, dass** die raue Oberfläche als
a) Plastikmaterialien, die auf einem 3D-Drucker erzeugt wurden oder
b) Hülsen, die mittels 3D-Drucks erzeugt und auf Plastikkämme gesteckt werden oder
c) Kämme, die an der Oberfläche aufgeraut wurden
vorliegt.

3. Verfahren nach Anspruch 1 oder 2, **dadurch gekennzeichnet, dass** zur Senkung der Polarität der wässrigen Lösung organische Lösungsmittel, vorzugsweise Alkohole, eingesetzt werden.

4. Verfahren nach Anspruch 3, **dadurch gekennzeichnet, dass** die organischen Lösungsmittel in einer Konzentration zwischen 5 Vol% und 90 Vol.%, vorzugsweise zwischen 30 Vol% und 75 Vol.%, eingesetzt werden.

5. Verfahren nach einem der Ansprüche 1 bis 4, **dadurch gekennzeichnet, dass** die wässrige Lösung zusätzlich
a) Salze und / oder
b) mindestens ein Detergenz und / oder
c) Aminoalkohole oder Substanzen zur Einstellung des pH-Wertes, wie TRIS enthält.

6. Verfahren nach Anspruch 5, **dadurch gekennzeichnet, dass**
a) die Salze in einer Konzentration zwischen 1 mM und 5M vorliegen, vorzugsweise von 5 mM bis 2M
b) das Detergenz in einer Konzentration zwischen 0,1 Vol % und 30 Vol %, vorzugsweise 1 bis 10 %, vorliegt
c) TRIS in einer Konzentration zwischen 1mM und 2M, bevorzugt 10mM bis 1M, vorliegt.

7. Verfahren nach einem der Ansprüche 1 bis 6, **dadurch gekennzeichnet, dass** weitere Zusätze, vorzugsweise eine Proteinase, enthalten sind.

8. Verfahren nach einem der Ansprüche 1 bis 7, **dadurch gekennzeichnet, dass** die feste Phase innerhalb der wässrigen Lösung beweglich ist.

9. Testkit zur Isolierung von Nukleinsäuren aus wässrigen, Nukleinsäure-enthaltenden Proben, umfassend mindestens eine Substanz zur Senkung der Polarität der wässrigen Lösung, mindestens eine feste Phase in Form von
a) Hülsen, die mittels 3D-Drucks erzeugt und auf Plastikkämme gesteckt werden oder
b) Kämme, die an der Oberfläche aufgeraut wurden
zur Anbindung der Nukleinsäuren mit rauer Oberfläche, wobei die Rauheit der Oberfläche durch Berühren oder durch Ansicht der Oberfläche erkennbar ist, sowie Elutionspuffer.

10. Verwendung von Materialien mit rauer Oberfläche zur Isolierung von Nukleinsäuren aus Nukleinsäure-enthaltenden Proben, wobei die Rauheit der Oberfläche durch Berühren oder durch Ansicht der Oberfläche erkennbar ist, zur Isolierung von Nukleinsäuren aus Nukleinsäure-enthaltenden Proben.

## Claims

1. A method for isolating nucleic acids from aqueous nucleic acid containing samples, **characterized in that** an aqueous solution, which comprises free nucleic acids or nucleic acids liberated by lysis, before or after reduction in polarity of the aqueous solution is contacted with a solid phase having a rough plastic surface, wherein the roughness of the surface is recognizable by touching or viewing the surface, and the nucleic acids precipitate on the solid phase, and are subsequently removed from this aqueous solution with the solid phase.

2. The method according to claim 1, **characterized in that** the rough surface exists as
a) plastic materials which have been generated on a 3D printer or
b) sleeves, which are generated by means of 3D printing and are put on plastic combs or
c) combs which were roughened on the surface.

3. The method according to claim 1 or 2, **characterized in that** for reducing polarity of the aqueous solution, organic solvents are used, preferably alcohols.

4. The method according to claim 3, **characterized in that** the organic solvents are used in a concentration between 5 vol.% and 90 vol.%, preferably between 30 vol.% and 75 vol.%.

5. The method according to any one of claims 1 to 4, **characterized in that** the aqueous solution in addition contains
a) salts and / or
b) at least one detergent and / or
c) amino alcohols or substances for adjusting the pH value such as TRIS.

6. The method according to claim 5, **characterized in that**
a) the salts exist in a concentration between 1 mM and 5M, preferably from 5 mM to 2M
b) the detergent exists in a concentration between 0.1 vol. % and 30 vol. %, preferably 1 to 10 %,
c) TRIS exists in a concentration between 1mM and 2M, preferably 10mM to 1M.

7. The method according to any one of claims 1 to 6, **characterized in that** further additives, preferably a proteinase, are included.

8. The method according to any one of claims 1 to 7, **characterized in that** the solid phase is movable within the aqueous solution.

9. A test kit for isolating nucleic acids from aqueous nucleic acid containing samples, comprising at least a substance for reducing polarity of the aqueous solution, at least one solid phase in the form of
a) sleeves which are generated by means of 3D printing and are put on plastic combs or
b) combs which were roughened on the surface
for binding the nucleic acids with rough surface, wherein the roughness of the surface is recognizable by touching or viewing the surface, as well as elution buffers.

10. A use of materials with rough surface for isolating nucleic acids from nucleic acid containing samples, wherein the roughness of the surface is recognizable by touching or viewing the surface, for isolating nucleic acids from nucleic acid containing samples.

## Revendications

1. Procédé d'isolation des acides nucléiques à partir des échantillons aqueux comportant des acides nucléiques, **caractérisé en ce qu'**une solution aqueuse comprenant des acides nucléiques libres ou libérés par lyse, avant ou après la réduction de la polarité de la solution aqueuse, est mise en contact avec une phase solide comprenant une surface rugueuse en matière plastique, dans lequel la rugosité de la surface est reconnaissable par toucher ou par vue de la surface, et les acides nucléiques précipitent à la phase solide, et ensuite sont éliminés de cette solution aqueuse avec la phase solide.

2. Procédé selon la revendication 1, **caractérisé en ce que** la surface rugueuse existe comme
a) des matières plastiques qui ont été généré sur une imprimante 3D ou
b) des manchons qui sont générés au moyen de l'impression 3D et sont mis sur des peignes plastiques ou
c) des peignes qui ont été rendu rugueux sur la surface.

3. Procédé selon la revendication 1 ou 2, **caractérisé en ce que** des solvants organiques, de préférence, des alcools, sont utilisés pour réduire la polarité de la solution aqueuse,

4. Procédé selon la revendication 3, **caractérisé en ce que** les solvants organiques sont utilisés dans une concentration entre 5 % en volume et 90 % en volume, de préférence, entre 30 % en volume et 75 % en volume.

5. Procédé selon l'une quelconque des revendications 1 à 4, **caractérisé en ce que** la solution aqueuse comporte en outre
a) des sels et / ou
b) au moins un détergent et / ou
c) des aminoalcools ou des substances pour ajuster la valeur pH, comme TRIS.

6. Procédé selon la revendication 5, **caractérisé en ce que**
a) les sels existent dans une concentration entre 1 mM et 5M, de préférence, de 5 mM à 2M
b) le détergent existe dans une concentration entre 0,1 vol. % et 30 vol. %, de préférence, 1 à 10 %,
c) TRIS existe dans une concentration entre 1mM and 2M, de préférence, 10mM à 1M.

7. Procédé selon l'une quelconque des revendications 1 à 6, **caractérisé en ce que** des additifs supplémentaires, de préférence, une protéinase, sont inclus.

8. Procédé selon l'une quelconque des revendications 1 à 7, **caractérisé en ce que** la phase solide est mobile dans la solution aqueuse.

9. Test kit pour l'isolation des acides nucléiques à partir des échantillons aqueux comportant des acides nucléiques, comprenant au moins une substance pour la réduction de la polarité de la solution aqueuse, au moins une phase solide sous forme de
a) manchons qui sont générés au moyen de l'impression 3D et sont mis sur des peignes plastiques ou
b) des peignes qui ont été rendu rugueux sur la surface
pour lier les acides nucléiques avec une surface rugueuse, dans lequel la rugosité de la surface est reconnaissable par toucher ou par vue de la surface, ainsi qu'un tampon d'élution.

10. Utilisation des matériaux avec une surface rugueuse pour l'isolation des acides nucléiques à partir des échantillons comportant des acides nucléiques, dans lequel la rugosité de la surface est reconnaissable par toucher ou par vue de la surface, pour l'isolation des acides nucléiques à partir des échantillons comportant des acides nucléiques.
